# EUROPÄISCHE PATENTSCHRIFT

(11) **EP 1 618 918 B1**
(45) Veröffentlichungstag und Bekanntmachung des Hinweises auf die Patenterteilung: **16.09.2015**
(21) Anmeldenummer: 05105557.2
(22) Anmeldetag: 22.06.2005
(51) Int. Cl.: A61N 1/05

(54) **Implantierbare Elektrode**
Implantable electrode
Electrode implantable

(30) Priorität: 20.07.2004 DE 102004035904
(43) Veröffentlichungstag der Anmeldung: 25.01.2006
(73) Patentinhaber: Biotronik CRM Patent AG, 6340 Baar (CH)
(72) Erfinder: Geistert, Wolfgang, 79618 Rheinfelden (DE); Kuttler, Marc, 12205 Berlin (DE)
(74) Vertreter: Galander, Marcus

(56) Entgegenhaltungen:
- WO-A1-2004/112891
- US-A- 4 011 861
- US-A- 4 273 137
- US-A1- 2002 035 388
- US-A1- 2004 098 108
- US-B1- 6 287 332
- US-B1- 6 463 334

## Beschreibung

Gegenstand der Beschreibung ist eine implantierbare Elektrode, die zumindest einen elektrischen Leiter mit einem proximalen Anschlussbereich für einen Impulsgenerator sowie zumindest eine über den Leiter mit dem Impulsgenerator verbindbare Arbeitselektrode umfasst.

Die Erfindung ist in Anspruch 1 definiert.

Elektroden der gattungsgemäßen Art sind aus dem Stand der Technik wohlbekannt. Sie sind Bestandteil zahlreicher funktionaler Elektrostimulationsgeräte (FES), die zur Elektrobehandlung von Nerven- oder Muskelzellen im diagnostischen oder therapeutischen Bereich eingesetzt werden. Implantatsysteme für die funktionale Elektrostimulation umfassen beispielsweise Herzschrittmacher mit einem Impulsgenerator zur künstlichen Anregung von Herzaktionen, der in einem körperverträglichen Gehäuse mit dazugehöriger elektronischer Schaltung und Energieversorgung untergebracht ist. Das Gehäuse besitzt eine Anschlussstelle, an der die Elektrode angeschlossen wird.

Der Begriff "Elektrode" bezeichnet in der Medizintechnik nicht nur die Übergangsstelle der elektrischen Energie nach physikalischer Definition, sondern bezieht sich auch auf die Leitung aus elektrischem Leiter samt umhüllender Isolation sowie alle weiteren funktionellen Elemente, die mit der Leitung fest verbunden sind. Im folgenden wird zu Klarstellungszwecken der eigentlich im physikalischen Sinne agierende Abschnitt der Elektrode, der die Übergangsstelle der elektrischen Energie umfasst, als "Arbeitselektrode" bezeichnet.

Die für den Zweck des Einsatzes geeigneten Materialien sollten eine ausreichende Bioresistenz und zudem eine gute Biokompatibilität besitzen. Es ist demnach sicherzustellen, dass eine langfristige Implantation im Körper - also in einer hochkorrosiven Umgebung - ohne nennenswerte Abbauprozesse abläuft und nicht zu einer unerwünschten immunologischen Reaktion führt. Für die Isolierung der elektrischen Leitung bieten sich biokompatible Kunststoffe auf Basis von Silikon, Polycarbonaten, Epoxysilan, Polyurethan, Polysulfone, Polyethylen und Polyester an. Die Arbeitselektroden wiederum, die als Ableit-, Stimulation- und Messelektroden ausgelegt sein können, sind im Allgemeinen auf Basis biokompatibler Metalle, wie Platin, Iridium Titan oder Gold, geformt.

Werkstoffe für den Einsatz in Implantaten sind dann akzeptabel, wenn das umgebene Gewebe - auch bei erhöhtem Allergierisiko - nur im geringen Maße mit unspezifischen Entzündungen auf mechanische Störeinflüsse und die Anwesenheit des Werkstoffs reagiert.

Es hat sich gezeigt, dass Gewebsbereiche, die mechanischen Störeinflüssen ausgesetzt sind, zu einer verstärkten immunologischen Reaktion oder Entzündungsreaktion neigen. Elektroden weisen derartige Bereiche unter anderem an den Anschlussstellen zum Gehäuse des Impulsgebers auf, da dort die Geometrien der Bauteile einem "mechanisch flexiblen Ausweichen" der Elektrode Grenzen setzen. Ein weiterer Bereich besonderer Beanspruchung liegt im Bereich der Arbeitselektroden, die ja zur Ausübung ihrer Funktion unmittelbar an dem zu stimulierenden Gewebe anliegen müssen. Auch hier kommt es immer wieder zu unerwünschten immunologischen und Entzündungsreaktionen des Körpers. Ferner müssen Elektroden häufig mit Ankerelementen oder Haltestrukturen versehen werden, um eine relative räumliche Lage der Arbeitselektrode zum zu behandelnden Gewebe sicherzustellen. Auch in den Bereichen der Haltestrukturen und Ankerelemente, die in sehr vielfältiger Art und Weise ausgestaltet sein können, treten in der Regel vermehrt immunologischen und Entzündungsreaktionen auf.

Der vorliegenden Erfindung liegt somit die Aufgabe zugrunde, die Verträglichkeit implantierter Elektroden die zumindest einen elektrischen Leiter mit einem proximalen Anschlussbereich für einen Impulsgenerator sowie zumindest eine über den Leiter mit dem Impulsgenerator verbindbare Arbeitselektrode umfassen, weiter zu verbessern.

Dies wird dadurch erreicht, dass die Elektrode zumindest einen Abschnitt aufweist, der elementares Magnesium enthält. Es hat sich überraschenderweise gezeigt, dass elementares Magnesium offenbar einen positiven Effekt auf das umgebende Gewebe ausübt und einer starken immunologischen und Entzündungsreaktionen des Körpers auf die Anwesenheit der Elektrode entgegen wirkt. Der dem Effekt zugrunde liegende Wirkungsmechanismus ist bisher nicht ergründet. Der gewünschte Effekt stellt sich nur ein, wenn das Magnesium zumindest kurzfristig in elementarer Form vorliegt - sei es als Reinelement oder als Legierung.

Vorzugsweise liegt das Magnesium in Form einer biodegradierbaren Magnesiumlegierung vor, die mindestens 50 Gew.%, vorzugsweise mindestens 70 Gew.%, besonders bevorzugt mindestens 85 Gew.% Magnesium enthält. Unter "Biodegradation" werden hydrolytische, enzymatische und andere stoffwechselbedingte Abbauprozesse im lebendem Organismus verstanden, die zu einer allmählichen Auflösung zumindest großer Teile der Legierung führen. Synonym wird häufig der Begriff Biokorrosion verwendet. Der Begriff Bioresorption umfasst zusätzlich die anschließende Resorption der Abbauprodukte.

Die Legierung ist vorzugsweise vom Typ WE, d.h. sie enthält Yttrium und Seltenerden als Legierungskomponenten. Die vorgenannten Legierungen lassen sich gut verarbeiten und haben bei in vitro Versuchen einen antiproliferativen Effekt auf glatte Muskelzellen des Menschen erkennen lassen. Sie sind damit besonders geeignet unerwünschten Reaktionen des Körpers auf die Anwesenheit der Elektrode vorzubeugen. Möglicherweise unterstützen oder verstärken die mit Zersetzung der Legierung einhergehenden Prozesse die antiproliferative Wirkung.

Besonders bevorzugt sind biodegradierbare Magnesiumlegierungen, die Seltenerdmetalle und Yttrium enthalten, wobei die Sammelbezeichnung ,Seltenerdmetall' für die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide bezeichnet werden, steht. Besonders bevorzugt weisen die Magnesiumlegierungen folgende Gewichtsanteile der Legierungskomponenten auf:
- Seltenerdmetalle 2.0 bis 5.0 Gew.% und/oder
- Yttrium 3.5 bis 4.5 Gew.% und/oder
- Neodym 1.5 bis 3.0 Gew.% und/oder
- Zirkonium 0.3 bis 1.0 Gew.% und/oder
- Aluminium < 0.5 Gew.%, insbesondere < 0.01 Gew.%, und/oder
- Rest < 0.5 Gew.%, insbesondere < 0.3 Gew.%,
wobei Magnesium den auf 100 Gew.% verbleibenden Gewichtsanteil an der Legierung einnimmt. Die genannten Magnesiumlegierungen zeigen ein günstiges Abbauverhalten, hohe Biokompatibilität der Legierung als auch der Abbauprodukte und besitzen für das Einsatzgebiet ausreichende mechanische Eigenschaften.

Nach einer bevorzugten Variante der vorgenannten Ausführungsform wird die spezifische Zusammensetzung der Magnesiumlegierung sowie seine Modifikation dahingehend vorgegeben, dass die Zersetzung unmittelbar nach Implantation einsetzt und so lange Aufrecht erhalten wird, bis zumindest bereichsweise die Elektrode mit Bindegewebe überzogen ist. Dieser Zeitraum sollte vorzugsweise zwischen 1 bis 90 Tage, insbesondere 3 bis 30 Tage, betragen. Der Umfang der Abbauprozesse ist von den am Implantationsort herrschenden Bedingungen abhängig. Eine zumindest bereichsweise Bedeckung bedeutet, dass zumindest die Bereiche der magnesiumhaltigen Abschnitte zu mehr als 50 % Ihrer Fläche mit Bindegewebe überzogen sind.

In stark strömender Gewebsumgebung muss eine höhere Freisetzungsrate für Magnesium und/oder seine Abbauprodukte zur Sicherstellung des gewünschten Effekts vorgegeben werden, während es in Gewebsumgebung mit geringer oder gar keiner Strömung einer geringeren Freisetzungsrate der genannten Komponenten bedarf, um die erwünschte Wirkung zu erzielen. Die Freisetzungsrate ist zudem von der Geometrie der Abschnitte und dem Gehalt der magnesiumhaltigen Abschnitte an Magnesium abhängig. Dementsprechend sind in der Praxis die magnesiumhaltigen Abschnitte der Elektrode individuell an die jeweils gewünschte Applikation anzupassen, um das gewünschte Freisetzungsverhalten zu erzielen. Bekannt ist, dass ein höherer Magnesiumgehalt zu einer Erhöhung der Zersetzungsgeschwindigkeit führt. Ferner ist bekannt, dass die Verarbeitung des Werkstoffs, z.B. durch Strangpressen, zu einer Veränderung des metallischen Gefüges führt, und damit auch die Zersetzung beeinflussbar ist.

Der Abschnitt kann ganz oder in Teilen aus Magnesium oder seinen Legierungen bestehen. Vorzugsweise erfolgt die Bereitstellung von Magnesium oder seiner Legierungen in Form von kleinen Metallpartikeln, die in eine biodegradierbare Matrix eingebettet werden. Mit Zersetzung der Matrix werden die Partikel freigesetzt und ebenfalls langsam abgebaut. Die Partikel haben vorzugsweise einen Durchmesser von 0,1 µm bis 500 µm, insbesondere 1 µm bis 50 µm. Als Matrix wird Hyaluronsäure nebst seiner Derivaten bevorzugt, da dieses Biopolymer sehr rasch abgebaut wird und eine ausgesprochen hohe Bioverträglichkeit aufweist.

Ein magnesiumhaltiger "Abschnitt" kann beliebige Geometrien einnehmen. Beispielsweise kann die Leitung der Elektrode, d.h. der elektrische Leiter mit umhüllenden Isolatormaterial, über seine gesamte Erstreckung mit Magnesium oder seinen Legierungen beschichtet werden. Denkbar ist auch, den Abschnitt als eigenständiges Strukturelement in die Elektrode zu integrierten, z.B. in Form eines an vorgegebener Stelle auf die Leitung aufgezogenen Rings aus dem Werkstoff. Besonders geeignet sind dabei Ausführungsformen, bei denen die magnesiumhaltigen Abschnitte am proximalen Ende der Elektrode, also benachbart zum Anschlussbereich des Impulsgenerators, angeordnet sind. Ebenso sind Anordnungen bevorzugt, bei denen die Abschnitte neben den Arbeitselektroden angeordnet sind. Schließlich wird nach einer weiteren bevorzugten Ausführungsform ein magnesiumhaltiger Abschnitt auf oder neben den Ankerelementen oder Haltestrukturen der Elektrode angeordnet. In den an die genannten Bereiche der Elektrode nach Implantation anliegenden Geweben kommt es erfahrungsgemäß vermehrt zu Gewebsirritationen. Die soeben beschriebenen bevorzugten Ausführungsformen der Abschnitte bestehen wiederum ganz oder in Teilen aus Magnesium oder seinen Legierungen.

Weiterhin ist vorteilhaft, wenn der magnesiumhaltige Abschnitt eine elektrisch aktive Oberfläche der Arbeitselektrode bedeckt. Dieser Bereich der Arbeitselektrode ist nach Implantation und Aufnahme seiner gewünschten Funktionalität besonders anfällig für immunologischen und Entzündungsreaktionen, die teils zu einer Vernarbung des Gewebes führen und damit nachhaltig die Funktionalität der Arbeitselektrode beeinträchtigen können. Es hat sich überraschenderweise gezeigt, dass die Anwesenheit von elementarem Magnesium nicht wesentlich die Funktionalität von Ableitelektroden, Stimulationselektroden und Messelektroden einschränkt und dabei einen positiven supressiven Effekt auf die immunologische Antwort des umgebenden Gewebes ausübt. Der magnesiumhaltige Abschnitt ist vorzugsweise nur wenige Mikrometer, insbesondere 10-100 µm dick. Der Abschnitt ist ferner vorzugsweise derart ausgelegt, dass er sich innerhalb von 1 h bis 120 h, insbesondere 12 h bis 36 h, zu mehr als 90 Gew.% zersetzt hat. Die soeben beschriebenen bevorzugten Ausführungsformen der Abschnitte bestehen wiederum ganz oder in Teilen aus Magnesium oder seinen Legierungen.

Die aktive Oberfläche der Elektrode hat vorzugsweise Kavitäten (Vertiefungen, Schlitze, Löcher), die mit Magnesium(legierung) ausgefüllt sind. Zusätzlich kann die gesamte aktive Oberfläche noch mit einem sehr dünnen Überzug aus Magnesium versehen sein. Dieser löst sich schnell auf und bewirkt in den ersten Tagen eine Linderung der Akutreaktionen. Anschließend wirkt das in den Kavitäten befindliche Magnesium mit geringerer Dosis über einen längeren Zeitraum (einige Wochen).

Die Erfindung wird nachfolgend anhand von Ausführungsbeispielen und dazugehörigen Zeichnungen näher erläutert. Die Ausführungsbeispiele, die nicht Gegenstand der Ansprüche sind, dienen nur als Beispiele und fallen nicht unter den Rahmen der gegenwärhgen Erfindung. Es zeigen:
- Fig. 1: ein Elektrostimulationsgerät in Form eines Schrittmachers mit einer erfindungsgemäßen Elektrode;
- Fig. 2: einen Schnitt durch die Elektrode im Bereich eines magnesiumhaltigen Abschnitts;
- Fig. 3: eine weitere Variante einer Elektrode mit einem Ankerelement und
- Fig. 4: eine weitere Variante einer Elektrode mit einer Haltestruktur.

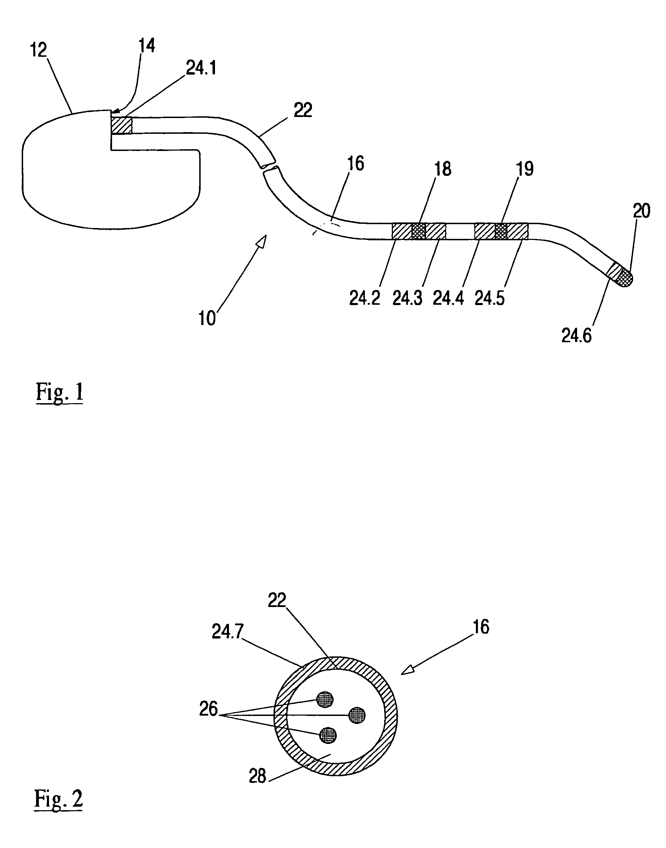

Fig. 1 zeigt in schematisierter Weise ein funktionales Elektrostimulationsgerät 10, das zur Elektrobehandlung von Nerven- oder Muskelzellen, insbesondere zur künstlichen Anregung von Herzaktionen, ausgelegt ist. Das Elektrostimulationsgerät 10 lässt sich in ein Gehäuse 12 und eine mit dem Gehäuse 12 an einer Anschlussstelle 14 verbundenen Elektrode 16 gliedern. Das Gehäuse 12 enthält für die Funktionalität des Elektrostimulationsgeräts 10 notwendige Komponenten, wie einen Impulsgenerator, elektrische Schaltungen und eine Energieversorgung.

Die Elektrode 16 weist an Ihrem proximalen, d. h. mit dem Gehäuse 12 verbundenen Ende geeignete, hier nicht näher ersichtliche Strukturen auf, die einen Anschluss an das Gehäuse 12 ermöglichen. Derartige Strukturen sind hinreichend aus dem Stand der Technik bekannt und haben im Zusammenhang mit der vorliegenden Erfindung keine nähere Bedeutung, so dass auf eine ausführliche Beschreibung derselben verzichtet wird.

Die Elektrode 16 besitzt drei Arbeitselektroden 18, 19, 20, wobei hierunter elektrisch leitfähige Strukturelemente verstanden werden, die eine Übergangsstelle für die elektrische Energie umfassen. Die beiden Arbeitselektroden 18, 19 sind beispielhaft als Ringelektroden ausgeführt und können aus einer Platin-Iridium-Legierung bestehen, während die distale Arbeitselektrode 20 einen halbkugelförmigen Kopf der Elektrode 16 bildet und aus einer Iridium-beschichteten Platin-Iridium-Legierung besteht. Die Arbeitselektroden 18, 19, 20 können als Ableit-, Stimulations- oder Messelektroden ausgelegt sein. Anzumerken ist, dass Material, Anzahl, Lage und Geometrie der Arbeitselektroden 18, 19, 20 in weitem Maße variiert werden können, ohne dass dies bedeutsame Auswirkungen auf den Gegenstand der vorliegenden Erfindung hat.

Die Elektrode 16 umfasst ferner eine Leitung 22, in der - hier nicht sichtbar dargestellt - ein oder mehrere elektrische Leiter verlaufen, wobei dieselben von einem Isolatormaterial, insbesondere auf Kunststoffbasis, umhüllt werden. Das Isolatormaterial sollte zumindest eine tolerierbare Biokompatibilität und ausreichende Bioresistenz besitzen. Hierzu geeignete Werkstoffe sind dem angesprochenen Fachmann hinreichend bekannt.

Die Elektrode 16 zeigt ferner sechs magnesiumhaltige Abschnitte 24.1 bis 24.6. Ein erster Abschnitt 24.1 ist benachbart zur Anschlussstelle 14 des Gehäuses 12, also im Bereich des proximalen Endes der Elektrode 16 angeordnet. Die magnesiumhaltigen Abschnitte 24.2 bis 24.5 fassen die ringförmigen Arbeitselektroden 18 und 19 ein. Der Abschnitt 24.6 grenzt schließlich unmittelbar an die Arbeitselektrode 20 am distalen Ende der Elektrode 16. Erfahrungsgemäß lassen sich Bereiche auf der Elektrode 16 bestimmen, die nach Implantation verstärkt Ausgangspunkt für Gewebsirritationen im umliegenden Gewebe sind. Die Abschnitte 24.1 bis 24.6 erstrecken sich nun insbesondere über diese Bereiche oder sind zumindest benachbart dazu angeordnet. Dem liegt die Erkenntnis zugrunde, dass Abschnitte 24.1 bis 24.6, die elementares Magnesium enthaltenen, zu einer signifikanten Minderung der Irritationen im umgebenden Gewebe führen können. Selbstverständlich kann auch die gesamte Leitung 22 mit einer elementares Magnesium enthaltenen Beschichtung oder einem anders gearteten Strukturelement überzogen werden kann.

Die magnesiumhaltigen Abschnitte 24.1 bis 24.6 können wir folgt realisiert werden:
1. Die Elektrode 16 wird maskiert mit einer wässrigen 40 %igen Hyaluronsäurelösung der Magnesiumpartikel zugesetzt sind, besprüht und anschließend getrocknet. Der Vorgang wird mehrfach wiederholt bis sich eine Schicht mit einer Dicke im Bereich von 0,1 bis 1 mm ausgebildet hat. Die Partikel weisen vorzugsweise einen Durchmesser von 0,1 µm bis 500 µm, insbesondere 1 µm bis 50 µm, auf und bestehen aus einer biodegradierbaren Magnesiumlegierung des Typs WE, konkret WE43, d. h. die Legierung enthält etwa 4 % Yttrium und etwa 3 % Seltenerden, insbesondere Neodym. Der kommerziell erhältliche Werkstoff WE43 wurde stranggepresst, durch übliche Verfahren in feine Partikel zerstäubt und gesiebt. Ein Gewichtsanteil der Magnesiumpartikel liegt nach dem Zusatz zur 40 %igen wässrigen Hyaluronsäurelösung bei etwa 1-5 %.
   Nach dieser ersten Variante werden demnach Magnesiumpartikel in eine biodegradierbare Matrix eingebettet und als Beschichtung auf die Elektrode 16 aufgebracht. Eine Haftung der Beschichtung kann gegebenenfalls durch Vorbehandlung der Zieloberflächen verbessert werden, Dies kann beispielsweise eine Plasmabehandlung oder das Aufbringen einer Haftvermittlerschicht beinhalten.
2. Nach einer zweiten Variante können die magnesiumhaltigen Abschnitte 24.1 bis 24.6 als eigenständige metallische Strukturelemente in die Elektrode 16 integriert werden. Im konkreten Fall werden die in Fig. 1 dargestellten magnesiumhaltigen Abschnitte 24.1 bis 24.6 als Hülsen auf die Leitung 22 aufgezogen und festgeklemmt oder die Hülsen sitzen in entsprechenden Aussparungen der Leitung 22. Die Hülsen haben eine Wandstärke von etwa 0,1 bis 1 mm. Die Hülse besteht aus Magnesium oder einer biodegradierbaren Magnesiumlegierung, insbesondere vom Typ WE.

Fig. 2 zeigt in stark schematisierter Weise einen Querschnitt durch die Leitung 22 der Elektrode 16 im Bereich eines magnesiumhaltigen Abschnitts 24.7. Die elektrische Leitung 22 umfasst insgesamt drei elektrische Leiter 26, die von einem Isolatormaterial 28 umhüllt werden. Nach außen hin schließt sich der magnesiumhaltige Abschnitt 24.7 an, der entweder aus als Beschichtung aus einer mit kleinen Magnesiumpartikeln versetzten, biodegradierbaren Matrix besteht oder massiv als Hülse aus einer biodegradierbaren Magnesiumlegierung ausgeführt wird.

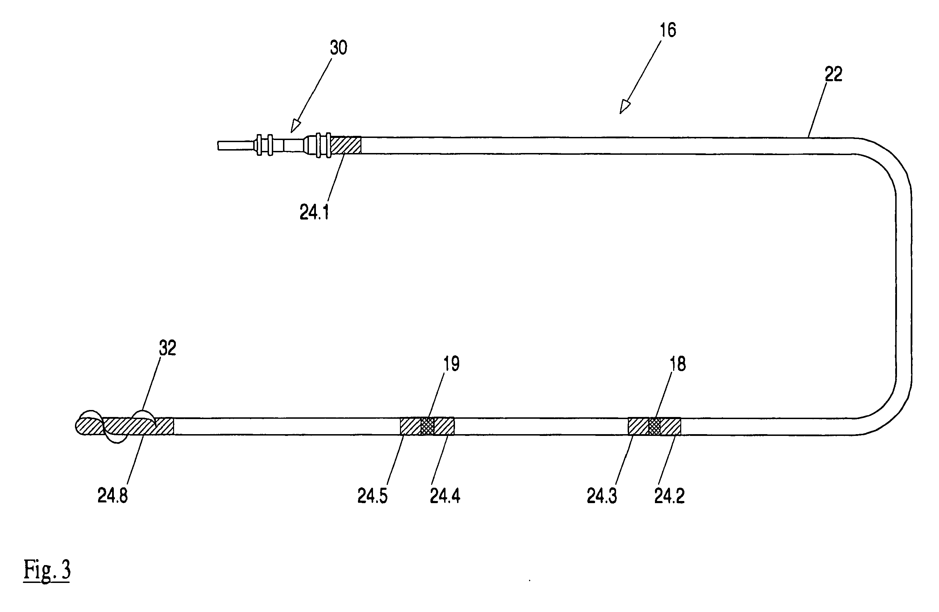

Fig. 3 zeigt eine weitere Elektrode 16, wobei funktionell gleich geartete Komponente der Elektrode 16 mit den gleichen Bezugszeichen der Fig. 1 versehen wurden. Der proximale Anschlussbereich 30 der Elektrode 16 trägt wiederum einen magnesiumhaltigen Abschnitt 24.1. Auch die beiden Arbeitselektroden 18, 19 werden von magnesiumhaltigen Abschnitten 24.2 bis 24.5 begrenzt. An ihrem distalen Ende trägt die Elektrode 16 ein Ankerelement 32, das schraubenförmig zur Spitze der Elektrode 16 zuläuft und eine Verankerung im umgebenden Gewebe nach bzw. bei Implantation dienen soll. Der gesamte distale Bereich der Elektrode 16, in dem das Ankerelement 32 angeordnet ist, wird von einem magnesiumhaltigen Abschnitt 24.8 bedeckt. Der Abschnitt 24.8 wird als Beschichtung aus einer biodegradierbaren Matrix mit zugesetzten Magnesiumpartikeln verwirklichbar. Hierzu wird auf die Variante 1 der bei Fig. 1 beschriebenen Vorgehensweise verwiesen.

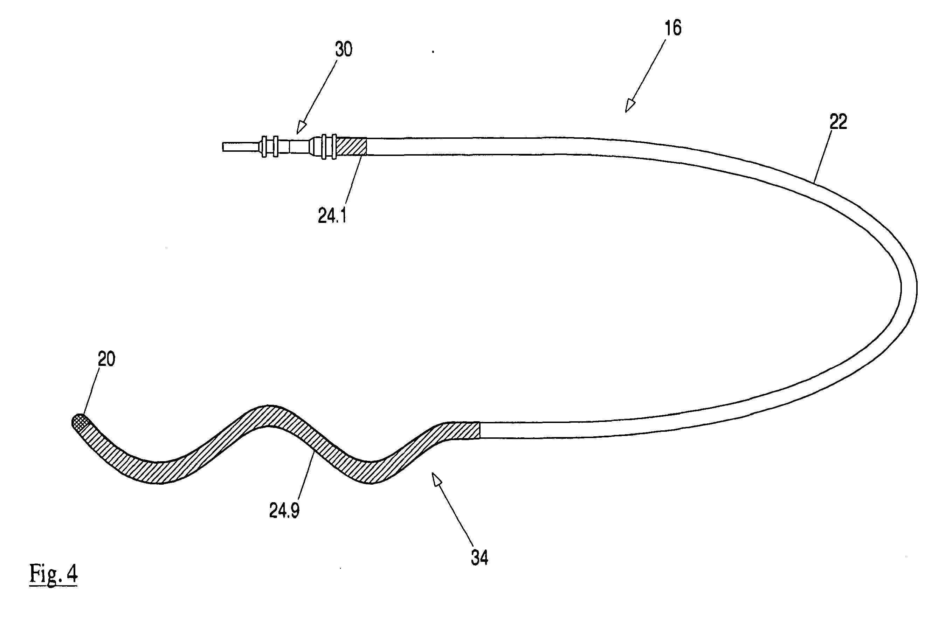

Fig. 4 zeigt eine weitere, alternative Ausführungsform der Elektrode 16, bei der eine Haltestruktur 34 die Elektrode 16 nach Implantation in gewünschter Lage halten soll. Die Haltestruktur 34 ist als wendelförmiger Abschnitt der Elektrode 16 ausgebildet und soll sich aufgrund der Formgebung am umgebenden Gewebe abstützen können. Der gesamte distale Bereich der Elektrode 16, der sich an die Arbeitselektrode 20 anschließt, und damit auch die Haltestruktur 34 wird vollständig von einem magnesiumhaltigen Abschnitt 24.9 abgedeckt. Der Abschnitt 24.9 lässt sich wiederum in Form einer Beschichtung, wie sie beispielhaft nach der Variante 1 der Fig. 1 darstellbar ist, bedeckt.

Die Erfindung ist in den folgenden Ansprüchen definiert:

## Patentansprüche

1. Implantierbare Elektrode (16), die zumindest einen elektrischen Leiter mit einem proximalen Anschlussbereich für einen Impulsgenerator sowie zumindest eine über den Leiter mit dem Impulsgenerator verbindbare Arbeitselektrode umfasst, wobei die Elektrode (16) zumindest einen Abschnitt (24.1 - 24.9), der elementares Magnesium enthält, umfasst, **dadurch gekennzeichnet, dass** das Magnesium in Form einer biodegradierbaren Legierung vorliegt, die mindestens 50 Gew.%, Magnesium enthält.

2. Elektrode (16) nach Anspruch 1, **dadurch gekennzeichnet, dass** die Magnesi-umlegierung vorzugsweise mindestens 70 Gew.%, besonders bevorzugt mindestens 85 Gew.% Magnesium enthält.

3. Elektrode (16) nach Anspruch 2, **dadurch gekennzeichnet, dass** die Magnesiumlegierung eine Legierung des Typs WE ist.

4. Elektrode (16) nach einem der Ansprüche 1 bis 3, **dadurch gekennzeichnet, dass** ein magnesiumhaltiger Abschnitt (24.1 - 24.9) am proximalen Ende der Elektrode (16) angeordnet ist.

5. Elektrode (16) nach einem der Ansprüche 1 bis 3, **dadurch gekennzeichnet, dass** ein magnesiumhaltiger Abschnitt (24.1 - 24.9) neben einer Arbeitselektrode (18, 19, 20) angeordnet ist.

6. Elektrode (16) nach einem der Ansprüche 1 bis 3, **dadurch gekennzeichnet, dass** ein magnesiumhaltige Abschnitt (24.1 - 24.9) eine elektrisch aktive Oberfläche der Arbeitselektrode (18, 19, 20) bedeckt.

7. Elektrode (16) nach Anspruch 6, **dadurch gekennzeichnet, dass** die elektrisch aktive Oberfläche der Arbeitselektrode Kavitäten (Vertiefungen, Schlitze, Löcher) aufweist, die mit Magnesium als Reinelement oder mit Magnesiumlegierung ausgefüllt sind.

8. Elektrode (16) nach einem der Ansprüche 1 bis 3, **dadurch gekennzeichnet, dass** ein magnesiumhaltiger Abschnitt (24.1 - 24.9) auf oder benachbart zu einer Haltestruktur (34) oder einem Ankerelement (32) der Elektrode (16) angeordnet ist.

9. Elektrode (16) nach einem der vorhergehenden Ansprüche, **dadurch gekennzeichnet, dass** der magnesiumhaltige Abschnitt 10 bis 100 µm dick ist.

10. Elektrode (16) nach einem der vorhergehenden Ansprüche, **dadurch gekennzeichnet, dass** die Bereitstellung der Magnesiumlegierungen in Form von kleinen Metallpartikeln, die in eine biodegradierbare Matrix eingebettet werden, erfolgt.

11. Elektrode (16) nach Anspruch 10, **dadurch gekennzeichnet, dass** die Partikel einen Durchmesser von 0,1 µm bis 500 µm, vorzugsweise 1 µm bis 50 µm, aufweisen.

12. Elektrode (16) nach Anspruch 10, **dadurch gekennzeichnet, dass** die Matrix Hyaluronsäure nebst seiner Derivaten ist.

## Claims

1. An implantable electrode (16), comprising at least one electrical conductor with a proximal connection area for an impulse generator and at least one working electrode that is connectable with the impulse generator via the conductor, wherein the electrode (16) contains at least one section (24.1 - 24.9) that contains elemental magnesium, **characterized in that** the magnesium is present in the form of a biodegradable alloy that contains at least 50% by weight of magnesium.

2. The electrode (16) according to Claim 1, **characterized in that** the magnesium alloy preferably contains at least 70% by weight, particularly preferred, at least 85% by weight of magnesium.

3. The electrode (16) according to Claim 2, **characterized in that** the magnesium alloy is an alloy of the WE type.

4. The electrode (16) according to one of Claims 1 through 3, **characterized in that** the magnesium-containing section (24.1 - 24.9) is located at the proximal end of the electrode (16).

5. The electrode (16) according to one of the Claims 1 through 3, **characterized in that** a magnesium-containing section (24.1 - 24.9) is located adjacent to the working electrode (18, 19, 20).

6. The electrode (16) according to one of the Claims 1 through 3, **characterized in that** a magnesium-containing section (24.1 - 24.9) covers an electrically active surface of the working electrode (18, 19, 20).

7. The electrode (16) according to Claim 6, **characterized in that** the electrically active surface of the working electrode has cavities (recesses, slots, holes) that are filled with magnesium in the form of the pure element or with magnesium alloy.

8. The electrode (16) according to one of the Claims 1 through 3, **characterized in that** the magnesium-containing section (24.1 - 24.9) is located on or adjacent to a retaining structure (34) or an anchoring element (32) of the electrode (16).

9. The electrode (16) according to one of the preceding Claims, **characterized in that** the magnesium-containing section is 10 to 100 µm thick.

10. The electrode (16) according to one of the preceding Claims, **characterized in that** the provision of the magnesium alloys takes place in the form of small metal particles that are embedded into a biodegradable matrix.

11. The electrode (16) according to Claim 10, **characterized in that** the particles have a diameter of 0.1 µm to 500 µm, preferably 1 µm to 50 µm.

12. The electrode according to Claim 10, **characterized in that** the matrix consists of hyaluronic acid in addition to its derivatives.

## Revendications

1. Electrode (16) implantable, qui comprend au moins un conducteur électrique avec une zone de connexion proximale pour un générateur d'impulsions ainsi qu'au moins une électrode de travail pouvant être connectée avec le générateur d'impulsions par l'intermédiaire du conducteur, où l'électrode (16) comprend au moins une partie (24.1 à 24.9) qui contient du magnésium sous forme d'élément, **caractérisée en ce que** le magnésium est présent sous la forme d'un alliage biodégradable qui contient au moins 50 % en poids de magnésium.

2. Electrode (16) selon la revendication 1, **caractérisée en ce que** l'alliage de magnésium contient au moins 70% en poids, de manière particulièrement préférée, au moins 85% en poids, de magnésium.

3. Electrode (16) selon la revendication 2, **caractérisée en ce que** l'alliage de magnésium est un alliage du type WE.

4. Electrode (16) selon l'une des revendications 1 à 3 **caractérisée en ce qu'**une partie contenant du magnésium (24.1 à 24.9) est disposée à l'extrémité proximale de l'électrode (16).

5. Electrode (16) selon l'une des revendications 1 à 3, **caractérisée en ce qu'**une partie contenant du magnésium (24.1 à 24.9) est disposée à côté de l'électrode de travail (18, 19, 20).

6. Electrode (16) selon l'une des revendications 1 à 3, **caractérisée en ce qu'**une partie contenant du magnésium (24.1 à 24.9) recouvre une surface électriquement active de l'électrode de travail (18, 19, 20).

7. Electrode (16) selon la revendication 6, **caractérisée en ce que** la surface électriquement active de l'électrode de travail présente des cavités (creux, fentes, trous) qui sont remplies avec du magnésium en tant qu'élément pur ou avec des alliages de magnésium.

8. Electrode (16) selon l'une des revendications 1 à 3, **caractérisée en ce qu'**une partie contenant du magnésium (24.1 à 24.9) est disposée sur ou au voisinage d'une structure support (34) ou d'un élément d'ancrage (32) de l'électrode (16).

9. Electrode (16) selon l'une des revendications précédentes, **caractérisée en ce que** la partie contenant du magnésium a une épaisseur de 10 à 100 µm.

10. Electrode (16) selon l'une des revendications précédentes, **caractérisée en ce que** la mise au point des alliages de magnésium est effectuée sous la forme de petites particules métalliques qui sont incorporées dans une matrice biodégradable.

11. Electrode (16) selon la revendication 10, **caractérisée en ce que** les particules présentent un diamètre de 0,1 µm à 500 µm, de préférence de 1 µm à 50 µm

12. Electrode (16) selon la revendication 10, **caractérisée en ce que** la matrice est de l'acide hyaluronique, en sus de ses dérivés.
